# EUROPÄISCHE PATENTSCHRIFT

(11) **EP 0 696 567 B1**
(45) Veröffentlichungstag und Bekanntmachung des Hinweises auf die Patenterteilung: **29.10.1997**
(21) Anmeldenummer: 95112004.7
(22) Anmeldetag: 31.07.1995
(51) Int. Cl.: C07C 67/54, C07C 69/01, C07C 69/06

(54) **Verfahren zur Reindestillation von Vinylformiat**
Process for the purification by distillation of vinyl formate
Procédé de purification par distillation du formiate de vinyle

(30) Priorität: 10.08.1994 DE 4428303
(43) Veröffentlichungstag der Anmeldung: 14.02.1996
(73) Patentinhaber: BASF AKTIENGESELLSCHAFT, 67056 Ludwigshafen (DE)
(72) Erfinder: Rühl, Thomas, Dr., D-67227 Frankenthal (DE); Heider, Marc, Dr., D-67433 Neustadt (DE); Henkelmann, Jochem, Dr., D-68165 Mannheim (DE); Rahn, Ralf-Thomas, Dr., D-68167 Mannheim (DE); Rust, Harald, D-67435 Neustadt (DE)

(56) Entgegenhaltungen:
- CH-A- 401 951

## Beschreibung

Die Erfindung betrifft ein verfahren zur Reindestillation von Vinylformiat, bei dem das nach der Synthese Ameisensäure, Acetaldehyd und Wasser enthaltende Vinylformiat destillativ von diesen Nebenprodukten getrennt wird.

Bei der Synthese von Vinylformiat aus Ameisensäure und Acetylen entsteht durch den thermischen Zerfall von intermediär entstandenem Ethylidenbisformiat auch Acetaldehyd. Acetaldehyd stellt bei Polymerisationen einen Kettenregler dar und führt zu kurzen Polymerisationscyclen und Kettenlängen. Für die Synthese von Polymerketten aus Vinylformiat muß der Acetaldehyd daher möglichst vollkommen aus der Reaktionsrohlösung entfernt werden. Versuche, durch eine einfache Destillation die Produkte voneinander zu trennen, führten trotz deutlicher Siedepunktunterschiede der Edukte (Acetaldehyd 21°C, Vinylformiat 47°C, Ameisensäure 101°C) zu Acetaldehyd-haltigen Vinylformiatfraktionen. Dies rührt daher, daß im Destillationssumpf Ethylidenbisformiat aus Ameisensäure und Vinylformiat entsteht, das wiederum Acetaldehyd bildet.

Dementsprechend liegt vorliegender Erfindung die Aufgabe zugrunde, ein Verfahren zur Reindestillation von Vinylformiat zu entwickeln.

Die Aufgabe wurde durch ein Verfahren der eingangs geschilderten Art gelöst, bei dem erfindungsgemäß das Vinylformiat in einer ersten Destillationsstufe von Ameisensäure und Wasser und in einer zweiten Stufe von Acetaldehyd getrennt wird.

Weitere Einzelheiten und Vorteile des Verfahrens nach der Erfindung sind in der nachfolgenden ausführlichen Beschreibung eines Ausführungsbeispiels enthalten.

Das aus der Syntheseanlage mit allen Nebenprodukten ausgetragene Vinylformiat wird kontinuierlich oder diskontinuierlich einer zweistufigen Destillation unterzogen. In der ersten Stufe werden Ameisensäure und Wasser abgetrennt. Die dazu eingesetzte Destillationskolonne ist beispielsweise eine Bodenkolonne oder eine Füllkörperkolonne mit 1 bis 15, vorzugsweise 5 bis 10 Böden, bei folgenden Betriebsparametern:
- Druckbereich:: 800 - 1020 mbar, vorzugsweise 950 - 1013 mbar
- Sumpftemperatur:: 100 - 130°C, vorzugsweise 110 - 125°C
- Kopftemperatur:: 30 - 60°C, vorzugsweise 35 - 50°C
- Rücklaufverhältnis:: 1:1 bis 10:1, vorzugsweise 2:1 bis 5:1

Acetaldehyd und Vinylformiat gehen dabei über Kopf in eine zweite Destillationskolonne, während im Sumpf der ersten Kolonne Ameisensäure und Wasser anfallen.

Die zweite Kolonne, die vorzugsweise ebenfalls eine Bodenkolonne mit 15 bis 45, vorzugsweise 20 bis 35 Böden ist, dient der Trennung von Acetaldehyd und Vinylformiat und arbeitet bei folgenden Betriebsparametern:
- Druckbereich:: 800 - 1020 mbar, vorzugsweise 950 - 1013 mbar
- Sumpftemperatur:: 80 - 120°C, vorzugsweise 95 - 110°C
- Kopftemperatur:: 10 - 25°C, vorzugsweise 15 - 21°C
- Seitenabzugstemperatur:: 35 - 80°C,, vorzugsweise 45 - 70°C
- Rücklaufverhältnis:: 1:1 bis 30:1, vorzugsweise 5:1 bis 20:1

Der Zulauf der zweiten Kolonne muß frei von Ameisensäure sein, da diese für die Entstehung von Acetaldehyd aus Vinylformiat verantwortlich ist.

Versuche im technischen Maßstab haben ergeben, daß nach dem erfindungsgemäßen Verfahren gewonnenes Vinylformiat eine Reinheit von 99,99 % aufweist.

### Beispiel:

Ein Rohaustrag der Zusammensetzung 55 % Vinylformiat, 40 % Ameisensäure und 5 % Acetaldehyd wird bei Normaldruck in einer Glockenbodenkolonne mit 11 Böden bei einer Verdamofertemperatur von 115°C destilliert. Dabei destillieren am Kopf Acetaldehyd und Vinylformiat im Verhältnis 7:93 ab. Im Sumpf fallen Ameisensäure und Wasser an.

Die Acetaldehyd/Vinylformiat-Fraktion wird anschließend in einer Glockenbodenkolonne mit 45 Böden und einer Verdampfertemperatur von 100°C destilliert. Im Sumpf fällt 99,99 %iges Vinylformiat bei einer Destillationsausbeute von 98 % über beide Stufen an.

## Patentansprüche

1. Verfahren zur Reindestillation von Vinylformiat, bei dem das nach der Synthese Ameisensäure, Acetaldehyd und Wasser enthaltende Vinylformiat destillativ von diesen Nebenprodukten getrennt wird, dadurch gekennzeichnet, daß das Vinylformiat in einer ersten Destillationsstufe von Ameisensäure und Wasser und in einer zweiten Stufe von Acetaldehyd getrennt wird.

2. Verfahren nach Anspruch 1, dadurch gekennzeichnet, daß die Destillationen bei Unterdruck durchgeführt werden.

## Claims

1. A process for distillative purification of vinyl formate by distilling as-synthesized vinyl formate to separate the vinyl formate from its synthesis by-products formic acid, acetaldehyde and water, characterized in that the vinyl formate is separated from formic acid and water in a first distillation stage and from acetaldehyde in a second stage.

2. A process as claimed in claim 1, characterized in that the distillations are carried out under reduced pressure.

## Revendications

1. Procédé d'épuration par distillation de formiate de vinyle, dans lequel le formiate de vinyle contenant, après la synthèse, de l'acide formique, de l'acétaldéhyde et de l'eau, est séparé par voie distillative de ces sous-produits, caractérisé par le fait que le formiate de vinyle est séparé de l'acide formique et de l'eau, dans une première étape de distillation, et, de l'acétaldéhyde, dans une deuxième étape.

2. Procédé selon la revendication 1, caractérisé par le fait que les distillations sont conduites sous vide.
